(19) Europäisches Patentamt / European Patent Office / Office européen des brevets

(11) **EP 4 481 096 B1**

(12) **EUROPEAN PATENT SPECIFICATION**

(45) Date of publication and mention
of the grant of the patent:
**06.05.2026   Bulletin 2026/19**

(21) Application number: **23780361.4**

(22) Date of filing: **27.03.2023**

(51) International Patent Classification (IPC):
*D04H 1/4242* (2012.01)   *D04H 1/43* (2012.01)
*D04H 1/4382* (2012.01)   *B01J 20/20* (2006.01)
*B01J 20/28* (2006.01)   *B01J 20/30* (2006.01)
*B01J 20/34* (2006.01)   *B01D 53/02* (2006.01)
*A61L 9/014* (2006.01)   *B01D 53/047* (2006.01)

(52) Cooperative Patent Classification (CPC):
**D04H 1/4242; A61L 9/014; B01D 53/02;
B01J 20/20; B01J 20/28; B01J 20/28023;
B01J 20/30; B01J 20/3416; D04H 1/43;
D04H 1/43835; D04H 1/43838; B01D 53/047;
B01D 2253/102; B01D 2253/25; B01D 2253/34**

(86) International application number:
**PCT/JP2023/012219**

(87) International publication number:
**WO 2023/190351 (05.10.2023 Gazette 2023/40)**

(54) **NONWOVEN FABRIC AND METHOD FOR PRODUCING THE SAME, ORGANIC SOLVENT RECOVERY METHOD USING THE SAME, AND ORGANIC SOLVENT RECOVERY APPARATUS**

VLIESSTOFF UND HERSTELLUNGSVERFAHREN DAFÜR, VERFAHREN ZUR RÜCKGEWINNUNG ORGANISCHER LÖSUNGSMITTEL DAMIT UND VORRICHTUNG ZUR RÜCKGEWINNUNG ORGANISCHER LÖSUNGSMITTEL

TISSU NON TISSÉ ET SON PROCÉDÉ DE FABRICATION, PROCÉDÉ DE RÉCUPÉRATION DE SOLVANT ORGANIQUE UTILISANT CE TISSU ET APPAREIL DE RÉCUPÉRATION DE SOLVANT ORGANIQUE

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priority: **31.03.2022   JP 2022058604**

(43) Date of publication of application:
**25.12.2024   Bulletin 2024/52**

(73) Proprietor: **Osaka Gas Chemicals Co., Ltd.
Osaka-shi, Osaka 550-0023 (JP)**

(72) Inventors:
• **MURAKAWA, Yuka**
**Osaka-shi, Osaka 550-0023 (JP)**
• **IMANISHI, Masachiyo**
**Osaka-shi, Osaka 550-0023 (JP)**

(74) Representative: **Murgitroyd & Company
165-169 Scotland Street
Glasgow G5 8PL (GB)**

(56) References cited:
WO-A1-2020/059819   WO-A1-2021/256324
JP-A- 2002 126 511   JP-A- H0 457 949
JP-A- H03 146 721   JP-A- S52 140 604
JP-U- H 081 677

## Description

### Technical Field

The present invention relates to a nonwoven fabric and a method for producing the nonwoven fabric, an organic solvent recovery method using the nonwoven fabric, and an organic solvent recovery apparatus.

### Background Art

Nonwoven fabrics with an activated carbon fiber are generally superior in deodorization for various odor gasses. Accordingly, they are useful as a material of deodorants that are used in various scenes, such as industries, household use, automobiles, and daily life materials. In particular, nonwoven fabrics are used for organic solvent recovery apparatuses to recover organic solvents that are generated, for example, in production processes in the printing industry, the electric industry, and the machine industry. To such organic solvent recovery apparatuses, for example, a step is applied in which an odor gas is fed to a packed tower packed with a nonwoven fabric to allow the nonwoven fabric to adsorb an organic solvent contained in the odor gas, and the organic solvent adsorbed on the nonwoven fabric is then desorbed from the nonwoven fabric to recover the organic solvent.

As a nonwoven fabric that is used as a deodorizing material, for example, Patent Document 1 discloses an activated carbon sheet formed by mixing activated carbon and a thermally fusible synthetic fiber as main components and partially melting the thermally fusible synthetic fiber to adhere the components. Patent Document 2 discloses a deodorizing material consisting of 10% by mass or more or 30% by mass of an acrylate-based fiber, 5% by mass or more or 30% by mass of an activated carbon fiber, and other fibers.

Document 3 discloses a nonwoven fabric comprising carbon fibers with a diameter of 15-120 $\mu$m, intended for removing solvents from gases by adsorption.

### Citation List

### Patent Document

Patent Document 1: Japanese Patent Laid-Open No. 2003-64563
Patent Document 2: Japanese Patent Laid-Open No. 2000-93493
Patent Document 3: PCT Patent Application WO 2021/256324 A1.

### Summary of Invention

### Technical Problem

Nonwoven fabric for odor gas adsorption is required to have adsorption performance to odor gasses, durability (tensile strength) as nonwoven fabrics, and thermal resistance. However, if the blended rate of a hot-melt fiber, which is mixed as a reinforcing fiber for an activated carbon fiber in a nonwoven fabric for odor gas adsorption, is high, the blended rate of the activated carbon fiber is relatively small, and the resulting nonwoven fabric for odor gas adsorption fails to have sufficient adsorption performance. If the blended rate of the hot-melt fiber is low, on the other hand, the resulting nonwoven fabric for odor gas adsorption suffers from the lowering of durability (tensile strength) as nonwoven fabrics. The hot-melt fiber has a low melting point, and thus has a problem of poor thermal resistance as well.

In Patent Document 1, a thermally fusible synthetic fiber, which is a low-melting-point fiber, is used together with activated carbon, and hence the thermal resistance is insufficient. In Patent Document 2, at most about 30% by mass of an activated carbon fiber is blended, and hence the adsorption performance to odor gasses is disadvantageously insufficient.

The present invention has been made in view of such problems, and an object of the present invention is to provide a nonwoven fabric having superior adsorption performance to odor gasses, superior durability (tensile strength), and high thermal resistance and a method for producing the nonwoven fabric, an organic solvent recovery method using the nonwoven fabric, and an organic solvent recovery apparatus.

### Solution to Problem

The present inventor has diligently studied to achieve the object. As a result, the present inventor has found that a nonwoven fabric containing a specific carbon fiber or carbon fiber precursor (A) and a specific fiber (B) at a specific blend ratio has superior adsorption performance to odor gasses, superior durability (tensile strength), and high thermal resistance, and eventually completed the present invention.

The present invention includes the following modes of implementation.

[1] A nonwoven fabric containing: a carbon fiber or carbon fiber precursor (A) each having an average fiber diameter of 10 $\mu$m or more and 30 $\mu$m or less, a tensile strength (1) of 40 N/mm$^2$ or more and 300 N/mm$^2$ or less, and an elongation percentage of 0% or more and 10% or less; and a fiber (B) having an average fiber diameter of 5 $\mu$m or more and 30 $\mu$m or less and a tensile strength (2) of 200 N/mm$^2$ or more and 600 N/mm$^2$ or less, provided that the tensile strength (2) is higher than the tensile strength (1), wherein a blend ratio between the carbon fiber or carbon fiber precursor (A) and the fiber (B) ((A):(B)) is 50:50 to 99:1 on mass basis, the carbon fiber or carbon fiber precursor (A) is an activated carbon fiber or an infusibilized fiber, and the fiber (B) is a carbon fiber or a carbon fiber precursor.

[2] The nonwoven fabric according to [1], wherein the activated carbon fiber is a pitch-based activated carbon fiber, and the activated carbon fiber has a specific surface area of 600 m$^2$/g or more and 2000 m$^2$/g or less.

[3] The nonwoven fabric according to [1] or [2], having a thermal resistance temperature of 200°C or more and 500°C or less.

[4] The nonwoven fabric according to any of [1] to [3], wherein the fiber (B) is an acrylic carbon fiber, a pitch-based carbon fiber, a phenolic-resin-based carbon fiber, an acrylic flame-resistant carbon fiber, a pitch-based flame-resistant carbon fiber, a phenolic-resin-based flame-resistant carbon fiber, an acrylic carbonized carbon fiber, a pitch-based carbonized carbon fiber, or a phenolic-resin-based carbonized carbon fiber.

[5] The nonwoven fabric according to any of [1] to [4], wherein the fiber (B) is a polyacrylonitrile-based flame-resistant carbon fiber or a pitch-based carbon fiber each differing from the carbon fiber or carbon fiber precursor (A).

[6] The nonwoven fabric according to any of [1] to [5], wherein the nonwoven fabric exhibits a deodorization rate of 90% or more for any of odor gasses of ammonia, toluene, acetaldehyde, methyl mercaptan, trimethylamine, and acetic acid as measured in the following odor gas elimination test:

(Odor gas elimination test)

in a room at 20°C or more and 25°C or less and 30%RH or more and 60%RH or less, 5 g of the nonwoven fabric to be subjected to measurement is put in a gas collection bag having a capacity of 10 L; subsequently, gas collection bag was charged with 3 L of air and sealed, and the gas collection bag was charged with any of the odor gasses in a predetermined initial gas concentration P and sealed, wherein the predetermined initial gas concentration P is 350 ppm for ammonia, 500 ppm for toluene, 100 ppm for acetaldehyde, 40 ppm for methyl mercaptan, 70 ppm for trimethylamine, and 100 ppm for acetic acid; the gas collection bag is left to stand for 30 minutes, and a concentration Q of ammonia, toluene, acetaldehyde, methyl mercaptan, trimethylamine, or acetic acid in the gas collection bag is then measured with a gas-detecting tube; and a deodorization rate for each of ammonia, toluene, acetaldehyde, methyl mercaptan, trimethylamine, and acetic acid is calculated from the following expression (1) with the initial gas concentration P and the concentration Q:

Deodorization rate (%) = $[(P - Q) / P] \times 100 \cdots$ (1)

[7] A method for producing the nonwoven fabric according to any of [1] to [6], wherein the carbon fiber (A) is an activated carbon fiber, and the method includes: a step of defibrating and mixing the activated carbon fiber and the fiber (B) to provide a nonwoven fabric.

[8] A method for producing the nonwoven fabric according to any of [1] to [6], wherein the carbon fiber precursor (A) is an infusibilized fiber, and the method includes: a step of defibrating and mixing the infusibilized fiber and the fiber (B) to provide a nonwoven fabric precursor; and an activation step of subjecting the nonwoven fabric precursor to activation treatment.

[9] An organic solvent recovery method including: an adsorption step of allowing the nonwoven fabric according to any of [1] to [6] to adsorb an organic solvent in an odor gas; a desorption step of desorbing the organic solvent from the nonwoven fabric with a desorbing gas, or a pressure-swing-adsorption-based desorption step of desorbing the organic solvent from the nonwoven fabric by depressurizing; and a recovery step of recovering the desorbed organic solvent.

[10] An organic solvent recovery apparatus including: an adsorbing unit that allows the nonwoven fabric according to any of [1] to [6] to adsorb an organic solvent in an odor gas; a desorbing unit that desorbs the organic solvent from the nonwoven fabric with a desorbing gas, or a pressure-swing-adsorption-based desorbing unit that desorbs the organic solvent from the nonwoven fabric by depressurizing; and a recovering unit that recovers the desorbed organic solvent.

Advantageous Effects of Invention

The present invention can provide a nonwoven fabric having superior adsorption performance to odor gasses, superior durability (tensile strength), and high thermal resistance and a method for producing the nonwoven fabric, an organic solvent recovery method using the nonwoven fabric, and an organic solvent recovery apparatus.

Description of Embodiments

The following describes embodiments to implement the present invention (hereinafter, referred to as "the present embodiment"), but the present invention is not limited to the description, and various modifications without departing from the scope can be made.

[Nonwoven Fabric]

The nonwoven fabric of the present embodiment contains: a carbon fiber or carbon fiber precursor (A) having an average fiber diameter of 10 $\mu$m or more and 30 $\mu$m or less, a tensile strength (1) of 40 N/mm$^2$ or more and 300 N/mm$^2$ or less, and an elongation percentage of 0% or more and 10% or less; and a fiber (B) having an average fiber diameter and 5 $\mu$m or more and 30 $\mu$m or less and a tensile strength (2) of 200 N/mm$^2$ or more and 600 N/mm$^2$ or less, provided that the tensile strength (2) is higher than the tensile strength (1), wherein the blend ratio between the carbon fiber or carbon fiber precursor (A) and the fiber (B) ((A):(B)) is 50:50 to 99:1 on mass basis.

(Carbon fiber or carbon fiber precursor (A))

The carbon fiber or carbon fiber precursor (A) according to the present embodiment has an average fiber diameter of 10 $\mu$m or more and 30 $\mu$m or less, a tensile strength (1) of 40 N/mm$^2$ or more and 300 N/mm$^2$ or less, and an elongation percentage of 0% or more and 10% or less. The carbon fiber according to the present embodiment is preferably an activated carbon fiber, which has been subjected to activation treatment and is superior in deodorization for various odor gases. For the activation treatment, for example, reference may be made to a method described later.

Herein, the average fiber diameter is measured with reference to JIS K1477. For the specific measurement method, reference may be made to Examples. Herein, the fiber diameter of a carbon fiber is the diameter of a perfect circle having the same area as the shape of the carbon fiber, and the average fiber diameter is the average value of fiber diameters measured for 30 or more filaments randomly picked out from a carbon fiber. The fiber diameter and average fiber diameter of the carbon fiber precursor, and the fiber diameter and average fiber diameter of the fiber (B) described later are defined in the same manner as those of the carbon fiber are defined.

The tensile strength is measured with reference to JIS K1477. For the specific measurement method, reference may be made to Examples.

The elongation percentage is measured with reference to JIS L1015. For the specific measurement method, reference may be made to Examples.

With the use of such a carbon fiber or carbon fiber precursor (A), a nonwoven fabric having superior adsorption performance to odor gasses, superior durability (tensile strength), and high thermal resistance can be obtained. One carbon fiber or carbon fiber precursor (A) may be used alone; alternatively, two or more carbon fibers or carbon fiber precursors (A) may be used in combination.

The average fiber diameter is preferably 11 $\mu$m or more and 25 $\mu$m or less, and more preferably 12 $\mu$m or more and 22 $\mu$m or less because a nonwoven fabric having more superior adsorption performance to odor gases, more superior durability (tensile strength), and higher thermal resistance is obtained. If the average fiber diameter is more than 30 $\mu$m, the number of filaments per unit mass is small, and hence the resulting nonwoven fabric tends to have lower strength as a whole.

A carbon fiber or carbon fiber precursor (A) having a tensile strength (1) of more than 300 N/mm$^2$ tends to have a smaller specific surface area, and thus to be poor in adsorption performance to odor gasses. A carbon fiber or carbon fiber precursor (A) having a tensile strength (1) of less than 40 N/mm$^2$ has lower strength, and hence tends to be powdered in a defibrator before the formation of a nonwoven fabric, complicating the formation of a nonwoven fabric. The tensile strength (1) is preferably 45 N/mm$^2$ or more and 285 N/mm$^2$ or less because a nonwoven fabric having more superior adsorption performance to odor gasses and more superior durability (tensile strength) is obtained.

The specific surface area of the carbon fiber or carbon fiber precursor (A) is preferably 600 m$^2$/g or more and 2000 m$^2$/g or less because a nonwoven fabric having even more superior adsorption performance to odor gasses, even more superior durability (tensile strength), and even higher thermal resistance is obtained. Herein, the specific surface area is one determined by means of a BET method with a nitrogen gas adsorption isotherm at liquid nitrogen temperature. For example, the specific surface area can be measured with a specific surface area/pore distribution analyzer (e.g., TriStar 3000 (product name) manufactured by Shimadzu Corporation). For the specific measurement method, reference may be made to Examples.

The average fiber length of the carbon fiber or carbon fiber precursor (A) is preferably 1 cm or more and 30 cm or less because a nonwoven fabric can be more easily produced. Herein, the fiber length is the longest direct distance between the ends of a filament of the carbon fiber or carbon fiber precursor, and the average fiber length is, for example, the average value of fiber lengths measured for 30 or more filaments randomly picked out from the fiber or precursor. The fiber length and average fiber length of the fiber (B) described later are defined in the same manner as those of the carbon fiber or carbon fiber precursor are defined.

Any fiber that can be obtained with a conventional known method can be used as the carbon fiber or carbon fiber precursor (A), as long as the average fiber diameter, tensile strength (1), and elongation percentage fall within the above ranges. The carbon fiber or carbon fiber precursor (A) is typically obtained by subjecting a starting raw material to infusibilization treatment, flame-proofing treatment, carbonization treatment, or activation treatment. The starting raw material is a rayon-based fiber, an acrylic fiber such as a polyacrylonitrile (PAN)-based fiber, a pitch-based fiber, or a phenolic-resin-based fiber. For infusibilization treatment, flame-proofing treatment, carbonization treatment, and activation treatment, known treatment methods can be used. For the treatment methods, for example, reference may be made to methods described later.

In the present embodiment, a desired carbon fiber or carbon fiber precursor (A) can be obtained through proper control of a starting raw material, infusibilization treatment, flame-proofing treatment, carbonization treatment, and activation treatment.

Examples of the shape of the carbon fiber or carbon fiber precursor (A) include, but are not limited to, chopped strands, a long fiber, a short fiber, a roving, filaments, a tow, and whiskers.

A fiber obtained by subjecting a pitch-based fiber as a starting raw material to infusibilization treatment (hereinafter, also referred to as an "infusibilized fiber") may be used as the carbon fiber or carbon fiber precursor (A). The infusibilized fiber at least has an average fiber diameter, tensile strength (1), and elongation percentage falling within the above ranges. If such an infusibilized fiber is used, it is preferred to obtain a nonwoven fabric precursor and then obtain a nonwoven fabric by subjecting the nonwoven fabric precursor to activation treatment.

(Fiber (B))

The fiber (B) according to the present embodiment has an average fiber diameter of 5 $\mu$m or more and 30 $\mu$m or less and a tensile strength (2) of 200 N/mm$^2$ or more and 600 N/mm$^2$ or less, provided that the tensile strength (2) is higher than the tensile strength (1). Any conventional known fiber can be used as the fiber (B), as long as the average fiber diameter and the tensile strength (1) fall within those ranges.

With the use of such a fiber (B), a nonwoven fabric having superior adsorption performance to odor gasses, superior durability (tensile strength), and high thermal resistance can be obtained. One fiber (B) may be used alone; alternatively, two or more fibers (B) may be used in combination.

The average fiber diameter is preferably 7 $\mu$m or more and 20 $\mu$m or less, and more preferably 5 $\mu$m or more and 15 $\mu$m or less because a nonwoven fabric having more superior adsorption performance to odor gases, more superior durability (tensile strength), and higher thermal resistance is obtained. If the average fiber diameter is more than 30 $\mu$m, the number of filaments per unit mass is small, and hence the resulting nonwoven fabric tends to have lower strength as a whole.

If the tensile strength (2) is less than 200 N/mm$^2$, the resulting nonwoven fabric may have lower durability. If the tensile strength (2) is more than 600 N/mm$^2$, the fiber (B) has difficulty in being tangled with the carbon fiber or carbon fiber precursor (A) in defibration, web formation with a carding machine, and bonding with a needle-punching method, thus complicating production of a nonwoven fabric. The tensile strength (2) is higher than the tensile strength (1) of the carbon fiber or carbon fiber precursor (A). If the tensile strength (2) is lower than the tensile strength (1), the resulting nonwoven fabric does not have improved tensile strength and tends to be poor in durability, even though the fiber (B) is mixed with the carbon fiber or carbon fiber precursor (A).

The tensile strength (2) is preferably 220 N/mm$^2$ or more and 580 N/mm$^2$ or less, and more preferably 230 N/mm$^2$ or more and 570 N/mm$^2$ or less because a nonwoven fabric having more superior adsorption performance to odor gases, more superior durability (tensile strength), and higher thermal resistance is obtained.

The elongation percentage is preferably 0% or more and 50% or less, and more preferably 0% or more and 30% or less because a nonwoven fabric having more superior adsorption performance to odor gases, more superior durability (tensile strength), and higher thermal resistance is obtained. With the use of the fiber (B) having an elongation percentage falling within that range, the fiber (B) can be more reliably inhibited from being powdered in defibration, web formation with a carding machine, and bonding with a needle-punching method, and the carbon fiber or carbon fiber precursor (A) and the fiber (B) tend to be tangled together in a preferable manner.

The average fiber length of the fiber (B) is preferably 1 cm or more and 30 cm or less because a nonwoven fabric can be more easily produced.

Any fiber obtained with a conventional known method can be used as the fiber (B), as long as the average fiber diameter and the tensile strength (2) fall within the above ranges. The fiber (B) is preferably a carbon fiber or a carbon fiber precursor. Examples of the fiber (B) include a rayon-based fiber, an acrylic fiber such as a polyacrylonitrile (PAN)-based fiber, a pitch-based fiber, and a phenolic-resin-based fiber, each being a starting raw material of the fiber (B); a carbon fiber obtained by subjecting any of those fibers as a starting raw material to flame-proofing treatment (hereinafter, also referred to as a "flame-resistant carbon fiber"); and a carbon fiber obtained by subjecting any of those fibers as a starting raw material to carbonization treatment (hereinafter, also referred to as a "carbonized carbon fiber"). Examples of the acrylic fiber, the pitch-based fiber, and the phenolic-resin-based fiber flame-resistant carbon fiber include an acrylic carbon fiber, a pitch-based carbon fiber, and a phenolic-resin-based carbon fiber. Examples of the flame-resistant carbon fiber include an acrylic flame-resistant carbon fiber, a pitch-based flame-resistant carbon fiber, and a phenolic-resin-based flame-resistant carbon fiber. Examples of the carbonized carbon fiber include an acrylic carbonized carbon fiber, a pitch-based carbonized carbon fiber, and a phenolic-resin-based carbonized carbon fiber.

In the present embodiment, a desired fiber (B) can be obtained through proper control of the types of a fiber and a starting raw material, flame-proofing treatment, and carbonization treatment.

Examples of the flame-proofing treatment include a method of passing the fiber through a flame-proofing furnace of hot-air circulation type at 220°C or more and 300°C or less for a passing time of 30 minutes or more and 100 minutes or less.

Examples of the carbonization treatment include a method of heating at 300°C or more and 900°C or less under anaerobic conditions. Different carbonization treatment times can be appropriately set for different raw materials and different facilities for carbonization. An example of carbonization treatment time is 15 minutes or more and 20 hours or less. The carbonization treatment is performed with known production equipment such as a rotary kiln and a conveyer furnace under a nitrogen atmosphere. Washing treatment, drying treatment, and so on may be performed after the carbonization. Known conditions can be employed for them without limitation.

Examples of the shape of the fiber (B) include, but are not limited to, chopped strands, a long fiber, a short fiber, a roving, filaments, a tow, and whiskers.

(Blend ratio between carbon fiber or carbon fiber precursor (A) and fiber (B))

The blend ratio between the carbon fiber or carbon fiber precursor (A) and the fiber (B) ((A):(B)) is 50:50 to 99:1 on mass basis. With the blend ratio falling within the range, a nonwoven fabric having superior adsorption performance to odor gasses, superior durability (tensile strength), and high thermal resistance is obtained. Furthermore, the nonwoven fabric is capable of simultaneously absorbing multiple odor gasses having opposite characteristics with higher absorption capacity and high absorption rates (high absorption ability). If the ratio of the carbon fiber or carbon fiber precursor (A) is less than 50% by mass, the resulting nonwoven fabric tends to have lower adsorption performance to odor gasses. If the ratio of the fiber (B) is less than 1% by mass, the resulting nonwoven fabric tends to have lower tensile strength.

The blend ratio between the carbon fiber or carbon fiber precursor (A) and the fiber (B) ((A):(B)) is preferably 50:50 to 95:5 on mass basis because a nonwoven fabric having more superior adsorption performance to odor gasses, more superior durability (tensile strength), and higher thermal resistance is obtained.

[Nonwoven Fabric]

Carbon fibers subjected to activation treatment have very large specific surface areas due to their fiber structure, and micropores are directly opening in their fiber surfaces. This results in high contact efficiency for odor gasses, and allows higher adsorption and desorption efficiencies than other adsorbing materials to be exerted. However, carbon fibers subjected to activation treatment have limited uses because of their shape. Nevertheless, nonwoven fabrics obtained by processing a carbon fiber as in the present embodiment are generally superior in durability (tensile strength), and widely applicable. Accordingly, nonwoven fabrics are used in various scenes, such as industries, household use, automobiles, and daily life materials, and particularly useful as a material of deodorants. In particular, nonwoven fabrics are preferable for organic solvent recovery apparatuses to recover organic solvents that are generated, for example, in production processes in the printing industry, the electric industry, and the machine industry.

The thickness and weight of the nonwoven fabric are not limited, and appropriate thickness and weight can be selected according to the specific mode of use, the structure of a deodorant, and the form of an organic solvent recovery apparatus. The dimensions can be appropriately adjusted according to the specific mode of use. One nonwoven fabric may be used alone; alternatively, two or more nonwoven fabrics may be used in combination.

The mode of use of the nonwoven fabric can be appropriately adjusted according to the specific mode of use such as using the nonwoven fabric in the form of a sheet, using the nonwoven fabric in a bent state, and using the nonwoven fabric in a wrapping and filling state.

The nonwoven fabric may have a multilayer structure as a combination of two or more dry layers. If the nonwoven fabric has a multilayer structure, the multilayer structure may be such that the layers have the same carbon fiber content, or such that the layers have different carbon fiber contents.

The thermal resistance temperature of the nonwoven fabric is preferably 200°C or more, and more preferably 300°C or more because the nonwoven fabric comes to have even higher thermal resistance. The upper limit is not limited and typically 500°C or less, and may be 450°C or less. Thus, the nonwoven fabric has high thermal resistance temperature, and is superior in thermal resistance. Herein, thermal resistance is evaluated with hot-pressing, and thermal resistance temperature is measured with a thermal analyzer (TG-DTA). For the specific measurement methods, reference may be made to Examples.

The nonwoven fabric can be used for adsorbing odor gases such as ammonia, toluene, acetaldehyde, methyl mercaptan, trimethylamine, and acetic acid. The nonwoven fabric has superior adsorption performance to these odor gasses. The nonwoven fabric exhibits a deodorization rate of 90% or more for any of odor gasses of ammonia, toluene, acetaldehyde, methyl mercaptan, trimethylamine, and acetic acid as measured in odor gas elimination test shown below. Preferably, all the deodorization rates are 93% or more. The nonwoven fabric has more superior adsorption performance to acetaldehyde, methyl mercaptan, and trimethylamine among those odor gasses. The nonwoven fabric exhibits a deodorization rate preferably of 95% or more, more preferably of 98% or more for any of those odor gasses. The upper limit of each deodorization rate is not limited, and preferably 100% or less. For specific odor gas elimination test, reference may be made to Examples.

(Odor gas elimination test)

In a room at 20°C or more and 25°C or less and 30%RH or more and 60%RH or less, 5 g of a nonwoven fabric to be subjected to measurement is put in a gas collection bag having a capacity of 10 L. Subsequently, gas collection bag was charged with 3 L of air and sealed, and the gas collection bag was charged with any of gasses of ammonia, toluene, acetaldehyde, methyl mercaptan, trimethylamine, and acetic acid in a predetermined initial gas concentration P and sealed. The initial gas concentration P is 350 ppm for ammonia, 500 ppm for toluene, 100 ppm for acetaldehyde, 40 ppm for methyl mercaptan, 70 ppm for trimethylamine, and 100 ppm for acetic acid. The gas collection bag is left to stand for 30 minutes, and the concentration Q of ammonia, toluene, acetaldehyde, methyl mercaptan, trimethylamine, or acetic acid in the gas collection bag is then measured with a gas-detecting tube. The deodorization rate for each of ammonia, toluene, acetaldehyde, methyl mercaptan, trimethylamine, and acetic acid is calculated from the following expression (1) with the initial gas concentration P and the concentration Q:

$$\text{Deodorization rate (\%)} = [(P - Q) / P] \times 100 \cdots (1)$$

[Method for Producing Nonwoven Fabric]

For example, the nonwoven fabric of the present embodiment can be produced with a method, wherein the fiber to be used as the carbon fiber (A) is an activated carbon fiber, and the method includes: a step of defibrating and mixing the activated carbon fiber and the fiber (B) to provide a nonwoven fabric.

The activated carbon fiber can be provided, for example, by subjecting a rayon-based fiber, an acrylic fiber such as a polyacrylonitrile (PAN)-based fiber, a pitch-based fiber, or a phenolic-resin-based fiber as a starting raw material to activation treatment. The activated carbon fiber may be provided, for example, by subjecting a starting raw material to infusibilization treatment, flame-proofing treatment, and/or carbonization treatment before activation treatment, and subjecting the treated product to activation treatment.

Known methods can be used for the activation treatment, and examples thereof include a gas activation method such as use of water vapor or carbon dioxide, and a chemical activation method such as zinc chloride activation and phosphoric acid activation.

Specific examples include a gas activation method in which a starting raw material is subjected to heat treatment in an activating gas such as water vapor and carbon dioxide at 500°C or more and 1000°C or less, and a chemical activation method in which a starting raw material is mixed with an activating agent such as phosphoric acid, zinc chloride, potassium hydroxide, and sodium hydroxide and subjected to heat treatment at 300°C or more and 800°C or less. The activated carbon fiber obtained through activation treatment may be directly used. The activated carbon fiber obtained through activation treatment may be removed of attached components, surface functional groups, and the like before use by washing with an acid or water and performing heat treatment.

The nonwoven fabric is obtained with an activated carbon fiber as the carbon fiber (A), for example, in the following manner: specific amounts of the activated carbon fiber and the fiber (B) are defibrated and mixed according to a conventional method to give a mixture, from which a web-like form is formed, and thereafter the fibers in the mixture are bonded to each other to give the nonwoven fabric. Specifically, the nonwoven fabric is obtained, for example, in the following manner: the activated carbon fiber (A) and the fiber (B) are defibrated into individual filaments of the fibers with a carding machine, the filaments are spread into a sheet-like form to give a web, and the fibers are bonded, for example, by means of needle punching, chemical bonding, thermal bonding, hydroentangling, or stitch bonding, to give the nonwoven fabric.

Because the homogeneity of the weight of the web in the width direction and the length direction has influence on the quality of the nonwoven fabric, it is preferred to use a bale opener and a blender that allow the activated carbon fiber (A) and the fiber (B) to be blended before being fed to a carding machine, and also to continuously adjust the feed rate in the width direction with the use of a volumetric feeder, which is an alternative to conventional weighing-pan feeders.

The defibrating and mixing are performed, for example, with an opening and picking machine or a hopper. These machines may be combined as appropriate.

Examples of methods for forming a web-like form include a wet-laying method, what is called an air-laying method, which is a method of dispersing a raw material in air for forming, and a carding method. Among them, the carding method is preferred because the nonwoven fabric is more easily obtained.

In the carding method, for example, the activated carbon fiber (A) and the fiber (B) are defibrated into individual filaments by combing through three rolls: a main cylinder roll, a worker roll, and a stripper roll, each covered with serrated metallic wires, to give a thin sheet-like web. The resulting web is laminated, for example, with a web laminator to give a weight preferably of 100 g/m$^2$ or more and 500 g/m$^2$ or less, more preferably of 200 g/m$^2$ or more and 400 g/m$^2$ or less, even more preferably of 250 g/m$^2$ or more and 350 g/m$^2$ or less.

A method with a needle punch (hereinafter, referred to as the "needle-punching method") is preferred as a method for bonding the fibers in the web together because the nonwoven fabric is more easily obtained and reduction in thermal resistance is inhibited. The needle-punching method is a method of piercing a web with many hooked needles (needles) and mechanically entangling fibers together. Specifically, a web is introduced between a stripper blade and a bed blade, and a needle board on which needles have been provided is moved up and down with a needle beam to allow the needles to penetrate through the web. Each needle is typically provided with six or more and nine or less barbs arranged to oppose the direction of penetration, and several filaments of fibers are hooked by the barbs and thereby tightened.

The nonwoven fabric may be produced with addition of an additive such as a dispersing agent, a defoaming agent, a hydrophilic agent, an antistatic agent, a polymer thickener, a release agent, an antifungal agent, and a fungicide, as necessary.

Alternatively, the nonwoven fabric of the present embodiment can be produced with a method, wherein the carbon fiber precursor (A) is an infusibilized fiber, which is obtained through infusibilization treatment, and the method includes: a step of defibrating and mixing the infusibilized fiber and the fiber (B) to provide a nonwoven fabric precursor; and an activation step of subjecting the nonwoven fabric precursor to activation treatment.

Known methods can be used for the infusibilization treatment. Examples of such methods include a method of subjecting the carbon fiber or carbon fiber precursor (A) or a starting raw material thereof to heat treatment and/or electron beam irradiation in the presence of an active gas such as oxygen, iodine, ozone, nitrogen dioxide, and bromine. For the starting raw material of the carbon fiber or carbon fiber precursor (A), reference may be made to the above description. The active gas may be used in combination with an inert gas such as a rare gas such as argon and nitrogen. Since air normally contains about 21 vol% of oxygen, the infusibilization treatment can be performed under an air atmosphere.

The temperature for the infusibilization treatment depends on the glass transition temperature and melting point of the carbon fiber or carbon fiber precursor (A) or a starting raw material thereof, and it is preferred to appropriately increase the temperature according to the reaction of oxidation. The temperature is preferably 100°C or more, more preferably 150°C or more, and even more preferably 200°C or more. The upper limit of the temperature is, for example, 400°C or less, and may be 350°C or less. The reaction time for the infusibilization treatment is typically 5 minutes or more and 24 hours or less.

The infusibilized fiber is obtained, for example, by oxidizing a pitch-based carbon fiber with the above method.

The nonwoven fabric precursor is obtained, for example, by defibrating and mixing the infusibilized fiber obtained through the above step and the fiber (B). For a method for obtaining the nonwoven fabric precursor, reference may be made to the aforementioned step of defibrating and mixing the carbon fiber (A) and the fiber (B) to provide a nonwoven fabric.

The nonwoven fabric is obtained, for example, by subjecting the nonwoven fabric precursor obtained through the above step to activation treatment. For a method of activation treatment, reference may be made to the above description.

To the nonwoven fabric, chemical adsorption performance may be imparted by performing application of chemical solution. Conventional known methods can be used for the application of chemical solution, and examples thereof include a method in which application by chemical solution impregnation, chemical solution spraying, or the like is followed by dehydration or drying.

[Organic Solvent Recovery Method and Organic Solvent Recovery Apparatus]

The organic solvent recovery method of the present embodiment includes: an adsorption step of allowing a nonwoven fabric to adsorb an organic solvent in an odor gas; a desorption step of desorbing the organic solvent from the nonwoven fabric with a desorbing gas, or a pressure-swing-adsorption (PSA)-based desorption step of desorbing the organic solvent from the nonwoven fabric by depressurizing; and a recovery step of recovering the desorbed organic solvent. The organic solvent recovery apparatus of the present embodiment includes: an adsorbing unit that allows a nonwoven fabric to adsorb an organic solvent in an odor gas; a desorbing unit that desorbs the organic solvent from the nonwoven fabric with a desorbing gas, or a PSA-based desorbing unit that desorbs the organic solvent from the nonwoven fabric by depressurizing; and a recovering unit that recovers the desorbed organic solvent. The organic solvent recovery method of the present embodiment may include the same step as in the known organic solvent recovery methods, except that the nonwoven fabric of the present embodiment is used as the nonwoven fabric. The organic solvent recovery apparatus of the present embodiment may have the same configuration as a known organic solvent recovery apparatus, except that the nonwoven fabric of the present embodiment is used as the nonwoven fabric.

In the adsorption step, for example, an odor gas containing an organic solvent is brought into contact with the nonwoven fabric to allow the nonwoven fabric to adsorb the organic solvent.

The organic solvent recovery apparatus includes, for example, an adsorption tank for bringing an odor gas and the nonwoven fabric into contact as the adsorbing unit. The odor gas is sent from an introduction line for odor gasses to the adsorption tank with a blower, and the nonwoven fabric is allowed to adsorb the organic solvent in the adsorption tank, and eventually the odor gas becomes a treated clean gas and is discharged from an exhaust line to the atmosphere.

The adsorption tank may contain an adsorbing material differing from the nonwoven fabric according to the present embodiment, as necessary. Examples of the adsorbing material include activated carbon, zeolite, silica gel, activated alumina, and porous organic compounds.

In the desorption step, the organic solvent is desorbed from the nonwoven fabric on which the organic solvent has been adsorbed with a desorbing gas such as water vapor, or by depressurizing on the basis of the PSA method.

In the case of desorption with a desorbing gas, for example, the desorbing unit in the organic solvent recovery apparatus includes an introduction line through which a desorbing gas is introduced to the adsorption tank. For example, water vapor is introduced from the introduction line, and by the action of the water vapor the organic solvent is desorbed from the nonwoven fabric on which the organic solvent has been adsorbed in the adsorption tank. In the case of desorption based on the PSA method, for example, a pressure-adjustable desorbing unit is included, and the organic solvent is desorbed by depressurizing. The desorbing pressure is typically equal to or higher than the vacuum pressure and equal to or lower than the atmospheric pressure.

The desorption in the desorption step is typically performed at a temperature as high as 130°C or more. Accordingly, the thermal resistance temperature of the nonwoven fabric is preferably 130°C or more and, more preferably 200°C or more, and even more preferably 300°C or more. The upper limit of the thermal resistance temperature is not limited, and typically 500°C or less, and may be 450°C or less.

In the recovery step, the desorbed organic solvent is recovered.

The recovering unit in the organic solvent recovery apparatus includes, for example, a condenser for recovering the desorbed organic solvent, and a recovery line for sending the desorbed organic solvent to the condenser. The desorbed organic solvent is, for example, liquified and condensed to be recovered in the condenser. The water vapor containing the condensed organic solvent is typically separated from the recovered organic solvent, and recovered as separated drainage.

Having superior adsorption performance to odor gasses, superior durability (tensile strength), and high thermal resistance, the nonwoven fabric according to the present embodiment is applicable to odor gasses containing various organic solvents. Examples of organic solvents include methylene chloride, chloroform, carbon tetrachloride, ethylene chloride, trichloroethylene, tetrachloroethylene, o-dichlorobenzene, m-dichlorobenzene, flon-112, flon-113, HCFC, HFC, propyl bromide, butyl iodide, acetic acid, methyl acetate, ethyl acetate, propyl acetate, butyl acetate, methyl methacrylate, diethyl carbonate, ethyl formate, diethyl ether, dipropyl ether, tetrahydrofuran, dibutyl ether, anisole, methanol, ethanol, isopropanol, n-butanol, 2-butanol, isobutanol, t-butanol, allyl alcohol, pentanol, heptanol, ethylene glycol, diethylene glycol, phenol, o-cresol, m-cresol, p-cresol, xylenol, acetaldehyde, acetone, methyl ethyl ketone, methyl isobutyl ketone, cyclohexanone, phorone, acetonitrile, acrylonitrile, n-hexane, isohexane, cyclohexane, methylcyclohexane, n-heptane, n-octane, n-nonane, isononane, decane, dodecane, undecane, tetradecane, decalin, benzene, toluene, m-xylene, o-xylene, p-xylene, ethylbenzene, 1,3,5-trimethylbenzene, n-methylpyrrolidone, dimethyl formamide, dimethyl acetamide, and dimethyl sulfoxide. One of these organic solvents or a mixture of one or more of them may be present.

Examples

The following specifically describes the present invention by showing examples and comparative examples, but the present invention is not limited to modes of those examples.

[Evaluation Method for Carbon Fiber or Carbon Fiber Precursor (A) and Fiber (B)]

The carbon fiber or carbon fiber precursor (A) and fiber (B) (hereinafter, simply referred to as the "fibers") were evaluated with methods shown below. Table 1 shows the evaluation results.

(1) Specific surface areas of fibers

The specific surface areas ($m^2$/g) of the fibers were measured on the basis of the BET method. Specifically, measurement was performed with a specific surface area/pore distribution analyzer (TriStar 3000 (product name) manufactured by Shimadzu Corporation).

(2) Average fiber diameters of fibers

The average fiber diameters ($\mu$m) of the fibers were measured with reference to JIS K1477. Specifically, the fibers were observed with a tabletop microscope (Miniscope (R) TM3000 (product name) manufactured by Hitachi High-Technologies Corporation) and subjected to image analysis to measure fiber diameter for 30 or more filaments randomly selected for each. The mean was calculated from the measurement results for each, and the means were designated as the average fiber diameters ($\mu$m) of the fibers.

(3) Tensile strengths of fibers

The tensile strengths (N/mm$^2$) of the fibers were measured with reference to JIS K1477. Specifically, the tensile strengths of the fibers were measured with a compact tabletop tester (EZ Test (product name) manufactured by Shimadzu Corporation) for 30 or more filaments randomly selected for each. The mean was calculated from the measurement results for each, and the means were designated as the tensile strengths (N/mm$^2$) of the fibers.

(4) Elongation percentages of fibers

The elongation percentages (%) of the fibers were measured with reference to JIS L1015. Specifically, the elongation percentages of the fibers were measured with a compact tabletop tester (EZ Test (product name) manufactured by Shimadzu Corporation) for 30 or more filaments randomly selected for each. The mean was calculated from the measurement results for each, and the means were designated as the elongation percentages (%) of the fibers.

[Example 1]

As shown in Table 1, 95% by mass of a pitch-based activated carbon fiber (A1) as the carbon fiber or carbon fiber precursor (A) and 5% by mass of a PAN (polyacrylonitrile)-based flame-resistant fiber (B1) (Pyromex (R) CPX2d51 (product name) manufactured by TEIJIN LIMITED) as the fiber (B) were defibrated with a defibrator and mixed to give a mixture. The mixture was continuously fed to a carding machine, subjected to lamination with a web laminator to give a weight of 300 g/m$^2$, and bonded with a needle-punching machine, providing a nonwoven fabric.

[Example 2]

As shown in Table 1, 50% by mass of a pitch-based activated carbon fiber (A1) as the carbon fiber or carbon fiber precursor (A), 45% by mass of a pitch-based activated carbon fiber (A2) as the carbon fiber or carbon fiber precursor (A), and 5% by mass of a PAN-based flame-resistant fiber (B1) (Pyromex (R) CPX2d51 (product name) manufactured by TEIJIN LIMITED) as the fiber (B) were defibrated with a defibrator and mixed to give a mixture. Thereafter, a nonwoven fabric was produced in the same manner as in Example 1.

[Example 3]

As shown in Table 1, 95% by mass of a pitch-based activated carbon fiber (A3) as the carbon fiber or carbon fiber precursor (A) and 5% by mass of a PAN-based flame-resistant fiber (B1) (Pyromex (R) CPX2d51 (product name) manufactured by TEIJIN LIMITED) as the fiber (B) were defibrated with a defibrator and mixed. Thereafter, a nonwoven fabric was produced in the same manner as in Example 1.

[Example 4]

As shown in Table 1, 50% by mass of a pitch-based activated carbon fiber (A1) as the carbon fiber or carbon fiber precursor (A) and 50% by mass of a PAN-based flame-resistant fiber (B1) (Pyromex (R) CPX2d51 (product name) manufactured by TEIJIN LIMITED) as the fiber (B) were defibrated with a defibrator and mixed to give a mixture. The mixture was continuously fed to a carding machine, subjected to lamination with a web laminator to give a weight of 100 g/m$^2$, and bonded with a needle-punching machine, providing a nonwoven fabric.

[Example 5]

As shown in Table 1, 95% by mass of a pitch-based activated carbon fiber (A4) as the carbon fiber or carbon fiber precursor (A) and 5% by mass of a PAN-based flame-resistant fiber (B1) (Pyromex (R) CPX2d51 (product name) manufactured by TEIJIN LIMITED) as the fiber (B) were defibrated with a defibrator and mixed. Thereafter, a nonwoven fabric was produced in the same manner as in Example 1.

[Example 6]

As shown in Table 1, a pitch-based fiber was subjected to infusibilization treatment to give an infusibilized fiber (A5) as the carbon fiber or carbon fiber precursor (A). Thereafter, 90% by mass of the infusibilized fiber and 10% by mass of a carbon fiber (B2) (DONACARBO (R) S-210F (product name) manufactured by Osaka Gas Chemicals Co., Ltd.) as the fiber (B) were defibrated with a defibrator and mixed to give a mixture. Subsequently the mixture was continuously fed to a carding machine, subjected to lamination with a web laminator to give a weight of 300 g/m$^2$, and bonded with a needle-punching machine, providing a nonwoven fabric precursor. The resulting nonwoven fabric precursor was activated to provide a nonwoven fabric.

[Comparative Example 1]

As shown in Table 1, a pitch-based activated carbon fiber (A2) was defibrated with a defibrator to give a defibrated product. Thereafter, the defibrated product was continuously fed to a carding machine, subjected to lamination with a web laminator to give a weight of 300 g/m$^2$, and bonded with a needle-punching machine, providing a nonwoven fabric.

[Comparative Example 2]

The physical properties of a nonwoven fabric disclosed in Example 1 in Japanese Patent Laid-Open No. 2000-93493 are shown in Table 1. Here, a common melting point of polyethylene terephthalate (PET) fibers, 250°C, was used as the thermal resistance temperature in Table 1, and a result disclosed in paragraph [0033] in Japanese Patent Laid-Open No. 2000-93493 was cited as the deodorization rate.

[Comparative Example 3]

As shown in Table 1, 40% by mass of a pitch-based activated carbon fiber (A1), 20% by mass of a hot-melt fiber (polyethylene fiber), and 40% by mass of a PET stretched fiber were mixed, and fused under a condition of 70°C or more and 250°C or less to provide a nonwoven fabric.

[Evaluation Methods for Nonwoven Fabrics]

(1) Thermal resistance (evaluation with hot-pressing)

Each of the nonwoven fabrics obtained in the examples and comparative examples was cut into a square of approximately 10 cm. Thereafter, the cut pieces of the nonwoven fabrics were hot-pressed from above with an iron for household use under conditions of 130°C or more and 150°C or less and 100 N or more. After the hot-pressing, air was blown through each nonwoven fabric, and the differential pressures before and after that was measured with a handy manometer differential pressure gauge (HP-21 (product name) manufactured by SIBATA SCIENTIFIC TECHNOLOGY LTD.) to evaluate whether the nonwoven fabric was deformed or not. If the pressure loss of the nonwoven fabric after the hot-pressing was less than ±10% of the pressure loss of the nonwoven fabric before the hot-pressing, the nonwoven fabric was determined as being not deformed, and in the case of ±10% or more, the nonwoven fabric was determined as being deformed. Table 2 shows the results. In Table 2, "A" indicates being not deformed, and "C" indicates being deformed.

(2) Thermal resistance temperature (evaluation with thermal analyzer)

Approximately 5 mg of each of the nonwoven fabrics obtained in the examples and comparative examples was placed in a non-sealed container made of aluminum, and, with use of a thermogravimetric-differential scanning calorimetric simultaneous analyzer (TG8120 (product name) manufactured by Rigaku Corporation), a TG-DTA curve was obtained by increasing the temperature to 800°C at a temperature increase rate of 10°C/min in air. At that time, the temperature at which a heat loss of 20% was observed was determined as the thermal resistance temperature (thermal decomposition temperature, °C). Table 2 shows the results.

(3) Tensile strength ratio

Each of the nonwoven fabrics obtained in the examples and comparative examples was cut into a piece of 100 mm in width and 150 mm in length, and a 25-mm cut was made from the center toward both edges in the longitudinal direction, setting a test width of 50 mm. The test piece was set on a compact tabletop tester (EZ Test manufactured by Shimadzu Corporation) and pulled in the longitudinal direction, and the maximum strength at rupture was determined as the tensile strength F (N/50 mm). The tensile strength ratio was calculated from an expression (2) with respect to 300 (g/m$^2$). Table 2 shows the results.

$$\text{Tensile strength ratio} = F \times (300 \: / \: \text{actual weight}) \cdots (2)$$

(4) Odor gas elimination test

The nonwoven fabrics obtained in the examples and comparative examples were subjected to gas deodorization test. Specifically, in a room at 20°C or more and 25°C or less and 30%RH or more and 60%RH or less, 5 g of a nonwoven fabric obtained in the examples was put in a gas collection bag having a capacity of 10 L. Subsequently, 3 L of air was charged with the gas collection bag and sealed, and any of gasses of ammonia, toluene, acetaldehyde, methyl mercaptan, trimethylamine, and acetic acid in a predetermined initial gas concentration P (350 ppm for ammonia, 500 ppm for toluene, 100 ppm for acetaldehyde, 40 ppm for methyl mercaptan, 70 ppm for trimethylamine, and 100 ppm for acetic acid) was then charged with the gas collection bag and sealed. The gas collection bag was left to stand for 30 minutes, and the concentration Q of ammonia, toluene, acetaldehyde, methyl mercaptan, trimethylamine, or acetic acid in the gas collection bag was then measured with a gas-detecting tube (manufactured by GASTEC CORPORATION). The deodorization rate for each of ammonia, toluene, acetaldehyde, methyl mercaptan, trimethylamine, and acetic acid was calculated from the following expression (1) with the initial gas concentration P and the concentration Q. Table 2 shows the results.

$$\text{Deodorization rate} \: (\%) = [(P-Q) \: / \: P] \times 100 \cdots (1)$$

[Table 1]

| Nonwoven fabric | | Example 1 | Example 2 | Example 3 | Example 4 | Example 5 | Example 6 | Comparative Example 1 | Comparative Example 2 | Comparative Example 3 |
|---|---|---|---|---|---|---|---|---|---|---|
| Carbon fiber or carbon fiber precursor (A) | | pitch-based activated carbon fiber (A1) | pitch-based activated carbon fiber (A1) | pitch-based activated carbon fiber (A3) | pitch-based activated carbon fiber (A1) | pitch-based activated carbon fiber (A4) | infusibilized fiber (A5) | pitch-based activated carbon fiber (A2) | pitch-based activated carbon fiber | pitch-based activated carbon fiber (A1) |
| Specific surface area | $m^2/g$ | 1000~1400 | 1000~1400 | 1400~1700 | 1000~1400 | 600~1000 | | 1400~1700 | 1000 | 1000~1400 |
| Average fiber diameter | $\mu m$ | 17~20 | 17~20 | 13~17 | 17~20 | 17~20 | 17~20 | 13~17 | | 17~20 |
| Tensile strength (1) | $N/mm^2$ | 140~280 | 140~280 | 100~250 | 140~280 | 140~280 | 50~100 | 50~110 | | 140~280 |
| Elongation percentage | % | 0~10 | 0~10 | 0~10 | 0~10 | 0~10 | 0~10 | 0~10 | | 0~10 |
| Blend ratio | % by mass | 95 | 50 | 95 | 50 | 95 | 90 | 100 | 5 | 40 |
| Carbon fiber or carbon fiber precursor (A) | | | pitch-based activated carbon fiber (A2) | | | | | | acrylate-based fiber | |
| Specific surface area | $m^2/g$ | | 1400~1700 | | | | | | | |
| Average fiber diameter | $\mu m$ | | 13~17 | | | | | | | |
| Tensile strength (1) | $N/mm^2$ | | 50~110 | | | | | | | |
| Elongation percentage | % | | 0~10 | | | | | | | |

EP 4 481 096 B1

13

| Nonwoven fabric | | Example 1 | Example 2 | Example 3 | Example 4 | Example 5 | Example 6 | Comparative Example 1 | Comparative Example 2 | Comparative Example 3 |
|---|---|---|---|---|---|---|---|---|---|---|
| Blend ratio | % by mass | | 45 | | | | | | 15 | |
| Fiber (B) | | PAN-based flame-resistant fiber (B1) | PAN-based flame-resistant fiber (B1) | PAN-based flame-resistant fiber (B1) | PAN-based flame-resistant fiber (B1) | PAN-based flame-resistant fiber (B1) | carbon fiber (B2) | | polyester fiber | hot-melt fiber + PET stretched fiber |
| Elongation percentage | % | 10~25 | 10~25 | 10~25 | 10~25 | 10~25 | 0~10 | | | |
| Tensile strength (2) | N/mm$^2$ | 240~350 | 240~350 | 240~350 | 240~350 | 240~350 | 300~560 | | | |
| Average fi-ber dia-meter | $\mu$m | 9~14 | 9~14 | 9~14 | 9~14 | 9~14 | 9~14 | | | |
| Blend ratio | % by mass | 5 | 5 | 5 | 50 | 5 | 10 | | 80 | 60(=20+40) |

EP 4 481 096 B1

[Table 2]

| Nonwoven fabric | | | Example 1 | Example 2 | Example 3 | Example 4 | Example 5 | Example 6 | Comparative Example 1 | Comparative Example 2 | Comparative Example 3 |
|---|---|---|---|---|---|---|---|---|---|---|---|
| Thermal resistance | | | A | A | A | A | A | A | A | A | C |
| Thermal resistance temperature | | °C | 400 | 400 | 400 | 400 | 400 | 400 | 400 | 250 | 130 |
| Tensile strength ratio | | | 4.3 | 2.4 | 4.3 | 4.5 | 2.2 | 1.5 | 1.0 | | |
| Deodorization rate | Ammonia | % | 99.8% | 99.4% | 94.5% | 99.8% | 98.1% | 99.6% | | 98.1% | |
| | Toluene | % | 100% | 98.2% | 100% | 100% | 100% | 100% | | 99.1% | |
| | Acetaldehyde | % | 100% | 100% | 100% | 100% | 100% | 100% | | 78.8% | |
| | Methyl mercaptan | % | 100% | 100% | 100% | 100% | 100% | 100% | | 82.5% | |
| | Trimethylamine | % | 100% | 100% | 100% | 100% | 100% | 100% | | 69.3% | |
| | Acetic acid | % | 100% | 98.1% | 96.1% | 99.5% | 98.0% | 98.2% | | 92.7% | |

The present application is based on a Japanese patent application filed on March 31, 2022 (Japanese Patent Application No. 2022-058604),

Industrial Applicability

The nonwoven fabric of the present invention has superior adsorption performance to odor gasses, superior durability (tensile strength), and high thermal resistance. Accordingly, the nonwoven fabric is used in various scenes, such as industries, household use, automobiles, and daily life materials, and particularly useful as a material of deodorants. In addition, the nonwoven fabric is preferable for organic solvent recovery apparatuses to recover organic solvents that are generated, for example, in production processes in the printing industry, the electric industry, and the machine industry.

**Claims**

1. A nonwoven fabric comprising:

   a carbon fiber or carbon fiber precursor (A) each having an average fiber diameter of 10 $\mu$m or more and 30 $\mu$m or less, a tensile strength (1) of 40 N/mm$^2$ or more and 300 N/mm$^2$ or less, and an elongation percentage of 0% or more and 10% or less; and
   a fiber (B) having an average fiber diameter of 5 $\mu$m or more and 30 $\mu$m or less and a tensile strength (2) of 200 N/mm$^2$ or more and 600 N/mm$^2$ or less, provided that the tensile strength (2) is higher than the tensile strength (1), wherein
   a blend ratio between the carbon fiber or carbon fiber precursor (A) and the fiber (B) ((A):(B)) is 50:50 to 99:1 on mass basis,
   the carbon fiber or carbon fiber precursor (A) is an activated carbon fiber or an infusibilized fiber, and
   the fiber (B) is a carbon fiber or a carbon fiber precursor.

2. The nonwoven fabric according to claim 1, wherein the activated carbon fiber is a pitch-based activated carbon fiber, and the activated carbon fiber has a specific surface area of 600 m$^2$/g or more and 2000 m$^2$/g or less.

3. The nonwoven fabric according to claim 1 or 2, having a thermal resistance temperature of 200°C or more and 500°C or less.

4. The nonwoven fabric according to any one of claims 1 to 3, wherein the fiber (B) is an acrylic carbon fiber, a pitch-based carbon fiber, a phenolic-resin-based carbon fiber, an acrylic flame-resistant carbon fiber, a pitch-based flame-resistant carbon fiber, a phenolic-resin-based flame-resistant carbon fiber, an acrylic carbonized carbon fiber, a pitch-based carbonized carbon fiber, or a phenolic-resin-based carbonized carbon fiber.

5. The nonwoven fabric according to any one of claims 1 to 4, wherein the fiber (B) is a polyacrylonitrile-based flame-resistant carbon fiber or a pitch-based carbon fiber each differing from the carbon fiber or carbon fiber precursor (A).

6. The nonwoven fabric according to any one of claims 1 to 5, wherein the nonwoven fabric exhibits a deodorization rate of 90% or more for any of odor gasses of ammonia, toluene, acetaldehyde, methyl mercaptan, trimethylamine, and acetic acid as measured in the following odor gas elimination test:
   (Odor gas elimination test)
   in a room at 20°C or more and 25°C or less and 30%RH or more and 60%RH or less, 5 g of the nonwoven fabric is put in a gas collection bag having a capacity of 10 L; subsequently, gas collection bag was charged with 3 L of air and sealed, and the gas collection bag was charged with any of the odor gasses in a predetermined initial gas concentration P and sealed, wherein the predetermined initial gas concentration P is 350 ppm for ammonia, 500 ppm for toluene, 100 ppm for acetaldehyde, 40 ppm for methyl mercaptan, 70 ppm for trimethylamine, and 100 ppm for acetic acid; the gas collection bag is left to stand for 30 minutes, and a concentration Q of ammonia, toluene, acetaldehyde, methyl mercaptan, trimethylamine, or acetic acid in the gas collection bag is then measured with a gas-detecting tube; and a deodorization rate for each of ammonia, toluene, acetaldehyde, methyl mercaptan, trimethylamine, and acetic acid is calculated from the following expression (1) with the initial gas concentration P and the concentration Q:

$$\texttt{Deodorization rate (\%) = [(P - Q) / P] × 100 ··· (1)}$$

**7.** A method for producing the nonwoven fabric according to any one of claims 1 to 6, wherein

the carbon fiber (A) is an activated carbon fiber, and
the method comprises:
a step of defibrating and mixing the activated carbon fiber and the fiber (B) to provide a nonwoven fabric.

**8.** A method for producing the nonwoven fabric according to any one of claims 1 to 6, wherein

the carbon fiber precursor (A) is an infusibilized fiber, and
the method comprises:

a step of defibrating and mixing the infusibilized fiber and the fiber (B) to provide a nonwoven fabric precursor; and
an activation step of subjecting the nonwoven fabric precursor to activation treatment.

**9.** An organic solvent recovery method comprising:

an adsorption step of allowing the nonwoven fabric according to any one of claims 1 to 6 to adsorb an organic solvent in an odor gas;
a desorption step of desorbing the organic solvent from the nonwoven fabric with a desorbing gas, or a pressure-swing-adsorption-based desorption step of desorbing the organic solvent from the nonwoven fabric by depressurizing; and
a recovery step of recovering the desorbed organic solvent.

**10.** An organic solvent recovery apparatus comprising:

an adsorbing unit that allows the nonwoven fabric according to any one of claims 1 to 6 to adsorb an organic solvent in an odor gas;
a desorbing unit that desorbs the organic solvent from the nonwoven fabric with a desorbing gas, or a pressure-swing-adsorption-based desorbing unit that desorbs the organic solvent from the nonwoven fabric by depressurizing; and
a recovering unit that recovers the desorbed organic solvent.

**Patentansprüche**

**1.** Ein Vliesstoff, der Folgendes beinhaltet:

eine Kohlenstofffaser oder einen Kohlenstofffaservorläufer (A), die jeweils einen durchschnittlichen Faserdurchmesser von 10 $\mu$m oder mehr und 30 $\mu$m oder weniger, eine Zugfestigkeit (1) von 40 N/mm$^2$ oder mehr und 300 N/mm$^2$ oder weniger und einen Dehnungsprozentsatz von 0 % oder mehr und 10 % oder weniger aufweisen; und
eine Faser (B), die einen durchschnittlichen Faserdurchmesser von 5 $\mu$m oder mehr und 30 $\mu$m oder weniger und eine Zugfestigkeit (2) von 200 N/mm$^2$ oder mehr und 600 N/mm$^2$ oder weniger aufweist, vorausgesetzt, dass die Zugfestigkeit (2) höher als die Zugfestigkeit (1) ist, wobei
ein Mischungsverhältnis zwischen der Kohlenstofffaser oder dem Kohlenstofffaservorläufer (A) und der Faser (B) ((A) : (B)) auf Massenbasis 50 : 50 bis 99 : 1 beträgt,
die Kohlenstofffaser oder der Kohlenstofffaservorläufer (A) eine Aktivkohlefaser oder eine unschmelzbar gemachte Faser ist und
die Faser (B) eine Kohlenstofffaser oder ein Kohlenstofffaservorläufer ist.

**2.** Vliesstoff gemäß Anspruch 1, wobei die Aktivkohlefaser eine pechbasierte Aktivkohlefaser ist und die Aktivkohlefaser eine spezifische Oberfläche von 600 m$^2$/g oder mehr und 2000 m$^2$/g oder weniger aufweist.

**3.** Vliesstoff gemäß Anspruch 1 oder 2, der eine Wärmebeständigkeitstemperatur von 200 °C oder mehr und 500 °C oder weniger aufweist.

**4.** Vliesstoff gemäß einem der Ansprüche 1 bis 3, wobei die Faser (B) eine Acrylkohlenstofffaser, eine pechbasierte Kohlenstofffaser, eine phenolharzbasierte Kohlenstofffaser, eine flammbeständige Acrylkohlenstofffaser, eine pechbasierte flammbeständige Kohlenstofffaser, eine phenolharzbasierte flammbeständige Kohlenstofffaser, eine karbonisierte Acrylkohlenstofffaser, eine pechbasierte karbonisierte Kohlenstofffaser oder eine phenolharzbasierte karbonisierte Kohlenstofffaser ist.

5. Vliesstoff gemäß einem der Ansprüche 1 bis 4, wobei die Faser (B) eine polyacrylnitrilbasierte flammbeständige Kohlenstofffaser oder eine pechbasierte Kohlenstofffaser ist, die sich jeweils von der Kohlenstofffaser oder dem Kohlenstofffaservorläufer (A) unterscheiden.

6. Vliesstoff gemäß einem der Ansprüche 1 bis 5, wobei der Vliesstoff eine Desodorierungsrate von 90 % oder mehr für beliebige der Geruchsgase von Ammoniak, Toluol, Acetaldehyd, Methylmercaptan, Trimethylamin und Essigsäure zeigt, wie im folgenden Geruchsgaseliminierungstest gemessen:

(Geruchsgaseliminierungstest)
in einem Raum bei 20 °C oder mehr und 25 °C oder weniger und 30 % RH oder mehr und 60 % RH oder weniger werden 5 g des Vliesstoffs in einen Gassammelbeutel mit einer Kapazität von 10 L gegeben; anschließend wurde der Gassammelbeutel mit 3 L Luft gefüllt und verschlossen, und der Gassammelbeutel wurde mit beliebigen der Geruchsgase in einer vorbestimmten anfänglichen Gaskonzentration P gefüllt und verschlossen, wobei die vorbestimmte anfängliche Gaskonzentration P 350 ppm für Ammoniak, 500 ppm für Toluol, 100 ppm für Acetaldehyd, 40 ppm für Methylmercaptan, 70 ppm für Trimethylamin und 100 ppm für Essigsäure beträgt; der Gassammelbeutel wird 30 Minuten stehen gelassen, und eine Konzentration Q von Ammoniak, Toluol, Acetaldehyd, Methylmercaptan, Trimethylamin oder Essigsäure im Gassammelbeutel wird dann mit einem Gasdetektionsrohr gemessen; und eine Desodorierungsrate für jedes von Ammoniak, Toluol, Acetaldehyd, Methylmercaptan, Trimethylamin und Essigsäure wird aus dem folgenden Ausdruck (1) mit der anfänglichen Gaskonzentration P und der Konzentration Q berechnet:

$$\text{Desodorierungsrate (\%)} = [(P - Q) / P] \times 100 \cdots (1).$$

7. Ein Verfahren zur Herstellung des Vliesstoffs gemäß einem der Ansprüche 1 bis 6, wobei

die Kohlenstofffaser (A) eine Aktivkohlefaser ist und
das Verfahren Folgendes beinhaltet:
einen Schritt des Defibrierens und Mischens der Aktivkohlefaser und der Faser (B), um einen Vliesstoff bereitzustellen.

8. Ein Verfahren zur Herstellung des Vliesstoffs gemäß einem der Ansprüche 1 bis 6, wobei

der Kohlenstofffaservorläufer (A) eine unschmelzbar gemachte Faser ist und
das Verfahren Folgendes beinhaltet:

einen Schritt des Defibrierens und Mischens der unschmelzbar gemachten Faser und der Faser (B), um einen Vliesstoffvorläufer bereitzustellen; und
einen Aktivierungsschritt des Unterziehens des Vliesstoffvorläufers einer Aktivierungsbehandlung.

9. Ein Rückgewinnungsverfahren für organische Lösungsmittel, das Folgendes beinhaltet:

einen Adsorptionsschritt des Ermöglichens, dass der Vliesstoff gemäß einem der Ansprüche 1 bis 6 ein organisches Lösungsmittel in einem Geruchsgas adsorbiert;
einen Desorptionsschritt des Desorbierens des organischen Lösungsmittels aus dem Vliesstoff mit einem desorbierenden Gas oder einen Desorptionsschritt auf Druckwechseladsorptionsbasis des Desorbierens des organischen Lösungsmittels aus dem Vliesstoff durch Entspannen; und
einen Rückgewinnungsschritt des Rückgewinnens des desorbierten organischen Lösungsmittels.

10. Eine Rückgewinnungsvorrichtung für organische Lösungsmittel, die Folgendes beinhaltet:

eine Adsorptionseinheit, die ermöglicht, dass der Vliesstoff gemäß einem der Ansprüche 1 bis 6 ein organisches Lösungsmittel in einem Geruchsgas adsorbiert;
eine Desorptionseinheit, die das organische Lösungsmittel aus dem Vliesstoff mit einem desorbierenden Gas desorbiert, oder eine Desorptionseinheit auf Druckwechseladsorptionsbasis, die das organische Lösungsmittel aus dem Vliesstoff durch Entspannen desorbiert; und

eine Rückgewinnungseinheit, die das desorbierte organische Lösungsmittel zurückgewinnt.

**Revendications**

1. Un tissu non tissé comprenant :

   une fibre de carbone ou un précurseur de fibre de carbone (A) ayant chacun(e) un diamètre de fibre moyen de 10 $\mu$m ou plus et de 30 $\mu$m ou moins, une résistance à la traction (1) de 40 N/mm$^2$ ou plus et de 300 N/mm$^2$ ou moins, et un pourcentage d'allongement de 0 % ou plus et de 10 % ou moins ; et
   une fibre (B) ayant un diamètre de fibre moyen de 5 $\mu$m ou plus et de 30 $\mu$m ou moins et une résistance à la traction (2) de 200 N/mm$^2$ ou plus et de 600 N/mm$^2$ ou moins, à condition que la résistance à la traction (2) soit supérieure à la résistance à la traction (1), où
   un rapport de mélange homogène entre la fibre de carbone ou le précurseur de fibre de carbone (A) et la fibre (B) ((A)/(B)) est de 50/50 à 99/1 sur une base en masse,
   la fibre de carbone ou le précurseur de fibre de carbone (A) est une fibre de carbone activé ou une fibre rendue infusible, et
   la fibre (B) est une fibre de carbone ou un précurseur de fibre de carbone.

2. Le tissu non tissé selon la revendication 1, où la fibre de carbone activé est une fibre de carbone activé à base de brai, et la fibre de carbone activé a une surface spécifique de 600 m$^2$/g ou plus et de 2 000 m$^2$/g ou moins.

3. Le tissu non tissé selon la revendication 1 ou la revendication 2, ayant une température de résistance thermique de 200 °C ou plus et de 500 °C ou moins.

4. Le tissu non tissé selon l'une quelconque des revendications 1 à 3, où la fibre (B) est une fibre de carbone acrylique, une fibre de carbone à base de brai, une fibre de carbone à base de résine phénolique, une fibre de carbone acrylique ignifuge, une fibre de carbone ignifuge à base de brai, une fibre de carbone ignifuge à base de résine phénolique, une fibre de carbone carbonisée acrylique, une fibre de carbone carbonisée à base de brai, ou une fibre de carbone carbonisée à base de résine phénolique.

5. Le tissu non tissé selon l'une quelconque des revendications 1 à 4, où la fibre (B) est une fibre de carbone ignifuge à base de polyacrylonitrile ou une fibre de carbone à base de brai qui diffèrent chacune de la fibre de carbone ou du précurseur de fibre de carbone (A).

6. Le tissu non tissé selon l'une quelconque des revendications 1 à 5, où le tissu non tissé présente un taux de désodorisation de 90 % ou plus pour n'importe lesquels d'entre des gaz odorants d'ammoniac, de toluène, d'acétaldéhyde, de méthylmercaptan, de triméthylamine, et d'acide acétique tel que mesuré dans l'essai d'élimination de gaz odorants suivant :

   (Essai d'élimination de gaz odorants)
   dans une pièce à 20 °C ou plus et 25 °C ou moins et 30 % HR ou plus et 60 % HR ou moins, 5 g du tissu non tissé sont placés dans un sac de collecte de gaz ayant une capacité de 10 L ; ensuite, le sac de collecte de gaz a été chargé avec 3 L d'air et scellé, et le sac de collecte de gaz a été chargé avec n'importe lesquels d'entre des gaz odorants dans une concentration de gaz initiale prédéterminée P et scellé, où la concentration de gaz initiale prédéterminée P est de 350 ppm pour l'ammoniac, de 500 ppm pour le toluène, de 100 ppm pour l'acétaldéhyde, de 40 ppm pour le méthylmercaptan, de 70 ppm pour la triméthylamine, et de 100 ppm pour l'acide acétique ; le sac de collecte de gaz est laissé au repos pendant 30 minutes, et une concentration Q de l'ammoniac, du toluène, de l'acétaldéhyde, du méthylmercaptan, de la triméthylamine, ou de l'acide acétique dans le sac de collecte de gaz est ensuite mesurée avec un tube de détection de gaz ; et un taux de désodorisation pour chacun(e) de l'ammoniac, du toluène, de l'acétaldéhyde, du méthylmercaptan, de la triméthylamine, et de l'acide acétique est calculé à partir de l'expression (1) suivante avec la concentration de gaz initiale P et la concentration Q :

$$\text{Taux de désodorisation (\%)} = [(P - Q)/P] \times 100 \cdots (1)$$

**7.** Un procédé de production du tissu non tissé selon l'une quelconque des revendications 1 à 6, où

la fibre de carbone (A) est une fibre de carbone activé, et
le procédé comprend :
une étape de défibrage et de mélange de la fibre de carbone activé et de la fibre (B) pour fournir un tissu non tissé.

**8.** Un procédé de production du tissu non tissé selon l'une quelconque des revendications 1 à 6, où

le précurseur de fibre de carbone (A) est une fibre rendue infusible, et
le procédé comprend :

une étape de défibrage et de mélange de la fibre rendue infusible et de la fibre (B) pour fournir un précurseur de tissu non tissé ; et
une étape d'activation consistant à soumettre le précurseur de tissu non tissé à un traitement d'activation.

**9.** Un procédé de récupération de solvant organique comprenant :

une étape d'adsorption consistant à permettre au tissu non tissé selon l'une quelconque des revendications 1 à 6 d'adsorber un solvant organique dans un gaz odorant ;
une étape de désorption consistant à désorber le solvant organique du tissu non tissé avec un gaz de désorption, ou une étape de désorption basée sur une adsorption modulée en pression consistant à désorber le solvant organique du tissu non tissé par dépressurisation ; et
une étape de récupération consistant à récupérer le solvant organique désorbé.

**10.** Un appareil de récupération de solvant organique comprenant :

une unité d'adsorption qui permet au tissu non tissé selon l'une quelconque des revendications 1 à 6 d'adsorber un solvant organique dans un gaz odorant ;
une unité de désorption qui désorbe le solvant organique du tissu non tissé avec un gaz de désorption, ou une unité de désorption basée sur une adsorption modulée en pression qui désorbe le solvant organique du tissu non tissé par dépressurisation ; et
une unité de récupération qui récupère le solvant organique désorbé.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2003064563 A
- JP 2000093493 A
- WO 2021256324 A1
- JP 2022058604 A